# EUROPÄISCHE PATENTANMELDUNG

(11) **EP 0 977 033 A2**
(43) Veröffentlichungstag der Anmeldung: **02.02.2000**
(21) Anmeldenummer: 99250253.4
(22) Anmeldetag: 29.07.1999
(51) Int. Cl.: G01N 33/00, G01N 31/22, G01N 33/68, G01N 33/52

(54) **Verfahren zur kosmetischen Bräunungsbestimmung einer Selbstbräunungszusammensetzung und Test-Kit**

(30) Priorität: 29.07.1998 DE 19834938
(71) Anmelder: Coty B.V., 2031 CC Haarlem (NL)
(72) Erfinder: Golz-Berner, Karin, 98000 Monaco (MC); Zastrow, Leonhard, 98000 Monaco (MC)
(74) Vertreter: Walter, Wolf-Jürgen

(57) **Zusammenfassung**

Die Erfindung betrifft ein Verfahren zur vorherigen Bestimmung eines Selbstbräunungsmittels bezogen auf unterschiedliche Hauttypen sowie ein Test-Kit zur Durchführung des Verfahrens. Erfindungsgemäß wird ein kosmetisches Selbstbräunungsmittel, das einen Gehalt an Dihydroxyaceton aufweist, auf wenigstens einem Testfeld dünn verrieben und nach einem Zeitraum von 5 Sekunden bis 5 Minuten die sich auf dem Testfeld ergebende Braunfärbung optisch ermittelt, wobei das Testfeld wenigstens eine Substanz trägt, die aus der Gruppe ausgewählt ist, die aus den Aminosäuren Gly, Ala, Val, Leu, Ile, Ser, Thr, Cys, Met, Asn, Lys, Asp, Gln, Glu, Phe, Tyr, Trp, His, Pro und den Purinen Adenin, Guanin, Xanthin und Hypoxanthin und Gemischen einzelner Verbindungen davon besteht.

## Beschreibung

Die Erfindung betrifft ein Verfahren zur vorherigen Bestimmung eines Selbstbräunungsmittels bezogen auf unterschiedliche Hauttypen sowie ein Test-Kit zur Durchführung des Verfahrens.

Es sind eine Vielzahl von Selbstbräunungszusammensetzungen bekannt und auf dem Markt, die als wesentliche Bräunungskomponente Dihydroxyaceton (1,3-Dihydroxypropan-2-on; im folgenden DHA) in unterschiedlichen Konzentrationen und Abmischungen mit anderen Komponenten enthalten. je nachdem welche Zusatzstoffe in derartigen kosmetischen Zusammensetzungen enthalten sind und in Abhängigkeit von der Konzentration des DHA werden unterschiedliche Farbtöne auf menschlicher Haut erzielt. Diese Farbtöne sind darüber hinaus auch von dem jeweiligen Hauttyp abhängig, d.h . vereinfacht von hellem, mittlerem oder dunklem Hauttyp.

Für den Anwender besteht das Problem, daß er bei der Vielzahl angebotener Selbstbräuner kaum voraussagen kann, mit welchem Produkt welcher Farbton erzielt werden kann, sofern nicht ein Vorversuch auf der Haut erfolgt und dann auf immer wieder das gleich Produkt mit konstanter Zusammensetzung zurückgegriffen wird.

Der Erfindung liegt die Aufgabe zugrunde, ein Verfahren zu entwickeln, mit dem die Brauntönung, die ein kosmetisches Selbstbräunungsmittel auf der Haut des Menschen erzielt, ohne vorherigen Hautkontakt weitgehend vorbestimmt werden kann.

Eine weitere Aufgabe der Erfindung besteht in der Bereitstellung einer Testkombination, mit der je nach Hauttyp des Anwenders eine Ermittlung der Hauteinfärbung des Selbstbräunungsmittels erreicht werden kann.

Erfindungsgemäß besteht das Verfahren zur kosmetischen Bräunungsbestimmung einer Selbstbräunungszusammensetzung, darin, daß man ein kosmetisches Selbstbräunungsmittel, das einen Gehalt an Dihydroxyaceton aufweist, auf wenigstens einem Testfeld, das sich auf einem Träger aus Papier oder einer aus natürlichen oder synthetischen Stoffen hergestellten Folie oder Platte befindet, dünn verreibt und nach einem Zeitraum von 5 Sekunden bis 5 Minuten die sich auf dem Testfeld ergebende Braunfärbung optisch ermittelt, wobei das Testfeld wenigstens eine Substanz trägt, die aus der Gruppe ausgewählt ist, die aus den Aminosäuren Gly, Ala, Val, Leu, Ile, Ser, Thr, Cys, Met, Asn, Lys, Asp, Gln, Glu, Phe, Tyr, Trp, His, Pro und den Purinen Adenin, Guanin, Xanthin und Hypoxanthin und Gemischen einzelner Verbindungen davon besteht.

Bevorzugte Aminosäuren sind solche mit basischer und hydrophiler Seitenkette, wie Histidin, Arginin und Lysin, jeweils in ihrer L-Form.

Ein bevorzugtes Purin ist Adenin (6-Aminopurin).

Eine bevorzugte Ausführungsform des Verfahrens besteht darin, daß das DHA enthaltende Selbstbräunungsmittel auf zwei Testfelder aufgetragen wird, wobei zuvor ein Testfeld mit Histidin und das andere Testfeld mit Adenin behandelt worden ist. An das Auftragen schließt sich ein Trocknungsprozeß an, z.B. bei etwa 20 bis 50 °C, vorzugsweise bei 20-40 °C.

Die Konzentration der Aminosäuren und der Purine beträgt dabei im allgemeinen 0,01 bis 1,5 Gew-%. Wenn die Konzentration unter 0,01 Gew-% liegt, wird die Bräunungsreaktion zu schwach. Konzentrationen über 1,5 Gew-% ergeben zu dunkle Tönungen und sind daher für die Erfindung nicht geeignet. Bevorzugte Konzentrationsbereiche der Substanzen liegen bei 0,05 bis 0,9 Gew-%.

Die Reinheit der eingesetzten Substanzen liegt im allgemeinen bei pharmazeutischer Reinheit, d.h. bei >99 %.

Nach dem Auftragen des DHA enthaltenden Selbstbräunungsmittels durch Aufstreichen oder Aufsprühen einer geringen Testmenge auf das Testfeld bildet sich im allgemeinen innerhalb weniger Sekunden, vorzugsweise 10 bis 30 Sekunden, eine Braunfärbung der behandelten Testfläche. Diese Braunfärbung ist stabil über einen langen Zeitraum (Stunden bis Tage) und kann vorzugsweise mit dem Auge oder wahlweise mit einem entsprechenden optischen Instrument erfaßt und im Vergleich mit dem eigenen Hauttyp des Anwenders und/oder mit Farbskalen ausgewertet werden.

Eine bevorzugte Ausführungsform der Erfindung besteht darin. daß zwei Testfelder mit den Substanzen markiert werden: ein Testfeld z.B. mit Histidin für einen dunklen Hauttyp und ein Testfeld mit Adenin für einen hellen Hauttyp. Der sich nach dem Auftragen des DHA enthaltenden Selbstbräunungsmittels ergebende Braunton entspricht dann etwa der Braunfärbung, die der Anwender mit hellem Hauttyp bzw. der Anwender mit dunklem Hauttyp bei Auftragen des Selbstbräunungsmittels auf seiner Haut erzielen würde.

Wegen unterschiedlicher DHA-Konzentrationen in den handelsüblichen Selbstbräunungsmitteln hilft das erfindungsgemäße Verfahren dem potentiellen Anwender eines beliebigen DHA enthaltenden Selbstbräunungsmittels, den sich bei Anwendung des Produktes ergebenden Farbton für seinen Hauttyp vorher zu ermitteln und danach seine Produktauswahl zu treffen.

Eine weitere Ausführungsform der Erfindung besteht darin, daß zwei oder drei Testfelder nebeneinander auf einem Träger jeweils mit der gleichen Substanz markiert werden, und die Testfelder mit unterschiedlichen Farbtönen vorgefärbt werden, wobei die Vorfärbung bestimmten Hautfärbungen entsprechen kann. Der sich beim Auftragen des DHA enthaltenden Selbstbräunungsmittels ergebende Mischfarbton entspricht dann dem Ergebnis für den jeweiligen Hauttyp.

Anstelle von DHA enthaltenden Selbstbräunungsmitteln können auch solche Bräunungsmittel eingesetzt werden, die DHA-Vorläufer enthalten, wie solche, bei dem der Wasserstoff einer oder beider OH-Gruppen durch einen Alkoxycarbonylrest oder Arylalkyloxycarbonylrest ersetzt ist, wobei die Reste 2 bis 25 Kohlenstoffatome haben können.

Das erfindungsgemäße Verfahren kann mit üblichen DHA enthaltenden Selbstbräunungsmitteln in unterschiedlichen Formulierungen durchgeführt werden. Übliche Formulierungen sind Cremes, Sprays, Gele, Lotionen, insbesondere Tagescremes, Sonnenschutzcremes, Körperlotionen, Sonnenschutzgele, Sonnenschutzsprays.

Derartige Präparate können weiterhin kosmetische Hilfs- und Trägerstoffe enthalten, wie sie üblicherweise in solchen Zubereitungen verwendet werden, z.B. Wasser, Konservierungsmittel, Vitamine, Farbstoffe, Radikalfänger, Verdickungsmittel, weichmachende Substanzen, feuchthaltende Substanzen, Duftstoffe, Alkohole, Polyole, Elektrolyte, Gelbildner, polare und unpolare Öle, Polymere, Copolymere, Emulgatoren, Wachse, Stabilisatoren.

Weiterhin können enthalten sein übliche wasser- und/oder öllösliche UVA- oder UVB-Filter oder beide sowie anorganische Pigmente auf Basis von Metalloxiden als Sonnenschutzmittel.

Die Erfindung betrifft weiterhin eine Testkombination (im folgenden Test-Kit) für Selbstbräunungsmittel auf Basis von Dihydroxyaceton, gekennzeichnet durch
ein Trägermaterial, bestehend aus Papier oder aus einer aus natürlichen oder synthetischen Stoffen hergestellten Folie oder Platte ;
ein auf dem Trägermaterial markiertes Feld, auf dem eine Substanz aufgetragen ist, die aus der Gruppe ausgewählt ist, die aus den Aminosäuren Gly, Ala, Val, Leu, Ile, Ser, Thr, Cys, Met, Asn, Lys, Asp, Gln, Glu, Phe, Tyr, Trp, His, Pro und den Purinen Adenin, Guanin, Xanthin und Hypoxanthin und Gemischen einzelner Verbindungen davon besteht, und die mit dem in dem Selbstbräunungsmittel enthaltenen Dihydroxyaceton oder Vorläufer von Dihydroxyaceton unter Ausbildung einer Braunfärbung reagiert.

Als Trägermaterial können Materialien verwendet werden, wie sie üblicherweise für Teststreifen eingesetzt werden. Dazu gehören verschiedene Papiere, Filme (z.B. nach DE 1598153) oder offene Filme (Z.B. nach DE 2910134), die ihrerseits auf einen Träger wie Folien, Papiere, Glas, Metall, Kunststoff-Tafeln usw. aufgebracht sein können. Die o.g. Filme können aus Filmbildnern wie Polyvinylester, Polyphenylacetale, Polyacrylester, Polymethacrylsäure, Polyacrylamide, Polyamide, Polystyren, Styren-Butadien-Mischpolymerisate und anderen filmbildenden natürlichen und synthetischen organischen Polymeren hergestellt werden, die vorzugsweise als wäßrige Dispersionen eingesetzt werden.

Als Trägermaterial kann jedoch auch saugfähiges Papier verwendet werden, da Probleme, wie sie bei quantitativen Tests infolge von Papierinhomogenitäten zu erwarten sind, bei dem erfindungsgemäßen Test nicht in dieser Form auftreten.

Mit der Erfindung ist es möglich, ein Testpapier bereitzustellen, bei dem der Anwender auf z.B. drei markierten Feldern seinen Hauttyp wie "hell", "mittel" oder "dunkel" auswählen kann und auf das für ihn zutreffende Testfeld das ebenfalls ausgewählte Selbstbräunungsmittel dünn aufträgt, z.B. durch leichtes Aufreiben oder Aufsprühen. Nach Abwarten der Reaktionszeit erfolgt dann der Farbvergleich, entweder direkt mit der eigen Haut oder über eine beiligende Farbtabelle.

Eine besonders vorteilhafte Ausführungsform des erfindungsgemäßen Test-Kit's besteht in einer kombinierten Form von Selbstbräunungsmittel-Probe zusammen mit einem mit den Testfeldern markierten Träger. Dabei kann die Probe in üblichen flachen, elastischen, fett- und wasserdichten versiegelten Probebehältern vorliegen, z.B. aus kaschierter Aluminimfolie, Plastikfolien usw. Auf diese Weise können z.B. Selbstbräunungsmittel-Proben mit unterschiedlichen DHA-Konzentrationen angeboten, auf voraussichtliche Hauttönung getestet und vom Anwender auf diese Weise je nach gewünschtem Bräunungston ausgewählt werden.

Eine weiter Ausführungsform des erfindungsgemäßen Test-Kit's besteht darin, daß neben einem oder mehreren Testfeldern eine Farbvergleichsskala für unterschiedliche Braunabstufungen aufgebracht ist.

Das Testfeld oder die Testfelder können zusätzlich mit einer transparenten oder nichttransparenten Folie abgedeckt sein, die vor dem Gebrauch abgezogen wird. Die Anwendung einer solchen Folie ist jedoch nicht zwingend, da normalerweise ein zusätzlicher Schutz des Testfeldes nicht erforderlich ist. Die auf dem Testfeld aufgetragene Subtanz ist im Normalfall wischfest, und eine Farbreaktion findet erst bei Vorhandensein von DHA auf dem Feld statt.

Die Erfindung soll nachstehend durch Beispiele näher erläutert werden. Alle Angaben erfolgen in Gewichtsprozent, sofern nichts anderes angegeben ist.

### Beispiel 1

Es wurde eine handelsüblich Selbstbräunungszusammensetzung mit einem Gehalt an DHA von 5,0 Gew-% eingesetzt.

Auf einem offenen Trägerfilm aus Polyvinylpropionat/Cellulose mit TiO₂-Anteilen wurde eine wäßrige Lösung von 0,2 Gew-% Adenin gleichmäßig aufgesprüht und im Trockenschrank bei 40 °C getrocknet.

Auf einen weiteren Trägerfilm wurde eine wäßrige Lösung von 0,2 Gew-% L-Histidin gleichmäßig aufgesprüht und im Trockenschrank bei 40 °C getrocknet.

Aus beiden Trägerfilmen wurden Stücke von etwa 2 cm² herausgeschnitten und auf einen Papierträger (Tiefdruckpapier) als markierte Testfläche I (Adenin) und II (Histidin) aufgebracht.

Das eingesetzte Selbstbräunungsmittel wurde dünn auf den beiden markierten Flächen mit dem Finger verrieben. Innerhalb von 10 Sekunden färbten sich die beiden Flächen in unterschiedlichen Brauntönen ein. Testfläche I zeigte dabei einen hellen Braunton und entsprach dem Ergebnis der Hautbräunung des DHA-Selbstbräunungsmittels bei Auftragen auf die Haut eines hellen Hauttyps. Testfläche II zeigte dagegen einen dunklen Braunton und entsprach dem Ergebnis der Hautbräunung des DHA-Selbstbräunungsmittels bei Auftragen auf die Haut eines dunklen Hauttyps.

## Patentansprüche

1. Verfahren zur kosmetischen Bräunungsbestimmung einer Selbstbräunungszusammensetzung, dadurch gekennzeichnet, daß man ein kosmetisches Selbstbräunungsmittel, das einen Gehalt an Dihydroxyaceton aufweist, auf wenigstens einem Testfeld, das sich auf einem Träger aus Papier oder einer aus natürlichen oder synthetischen Stoffen hergestellten Folie oder Platte befindet, dünn verreibt und nach einem Zeitraum von 5 Sekunden bis 5 Minuten die sich auf dem Testfeld ergebende Braunfärbung optisch ermittelt , wobei das Testfeld wenigstens eine Substanz trägt, die aus der Gruppe ausgewählt ist, die aus den Aminosäuren Gly, Ala, Val, Leu, Ile, Ser, Thr, Cys, Met, Asn, Lys, Asp, Gln, Glu, Phe, Tyr, Trp, His, Pro und den Purinen Adenin, Guanin, Xanthin und Hypoxanthin und Gemischen einzelner Verbindungen davon besteht.

2. Verfahren nach Anspruch 1, dadurch gekennzeichnet, daß eine Selbstbräunungszusammensetzung mit einem Gehalt an Dihydroxyaceton im Bereich von 0,5 bis 7 Gew-% eingesetzt wird.

3. Verfahren nach Anspruch 1, dadurch gekennzeichnet, daß auf dem Testfeld Histidin aufgetragen ist.

4. Verfahren nach Anspruch 1, dadurch gekennzeichnet, daß auf dem Testfeld Arginin aufgetragen ist.

5. Verfahren nach Anspruch 1, dadurch gekennzeichnet, daß auf dem Testfeld Adenin aufgetragen ist.

6. Verfahren nach Anspruch 1, dadurch gekennzeichnet, daß die sich auf dem Testfeld ergebende Braunfärbung mit dem Auge optisch ermittelt wird.

7. Verfahren nach Anspruch 1, dadurch gekennzeichnet, daß die sich auf dem Testfeld ergebende Braunfärbung mit einer vorgegebenen Vergleichsskala von Brauntönen verglichen wird.

8. Verfahren nach Anspruch 1, dadurch gekennzeichnet, daß die Substanz in einer Konzentration von 0,01 bis 1,5 Gew-% aufgetragen und danach einem Trocknungsprozeß unterworfen wird.

9. Verfahren nach Anspruch 1, dadurch gekennzeichnet, daß eine Selbstbräunungszusammensetzung mit einem Vorläufer von Dihydroxyaceton eingesetzt wird, bei dem der Wasserstoff einer oder beider OH-Gruppen durch einen Alkoxycarbonylrest oder Arylalkyloxycarbonylrest ersetzt ist, wobei die Reste 2 bis 25 Kohlenstoffatome haben können.

10. Kosmetisches Test-Kit für Selbstbräunungsmittel auf Basis von Dihydroxyaceton, gekennzeichnet durch
ein Trägermaterial, bestehend aus Papier oder aus einer aus natürlichen oder synthetischen Stoffen hergestellten Folie oder Platte ;
ein auf dem Trägermaterial markiertes Feld, auf dem eine Substanz aufgetragen ist, die aus der Gruppe ausgewählt ist, die aus den Aminosäuren Gly, Ala, Val, Leu, Ile, Ser, Thr, Cys, Met, Asn, Lys, Asp, Gln, Glu, Phe, Tyr, Trp, His, Pro und den Purinen Adenin, Guanin, Xanthin und Hypoxanthin und Gemischen einzelner Verbindungen davon besteht, und die mit dem in dem Selbstbräunungsmittel enthaltenen Dihydroxyaceton oder Vorläufer von Dihydroxyaceton unter Ausbildung einer Braunfärbung reagiert.

11. Test-Kit nach Anspruch 10, dadurch gekennzeichnet, daß auf dem Trägermaterial zwei Felder markiert sind, die unterschiedliche Substanzen aus der Gruppe der Aminosäuren und der Purine oder aus beiden Gruppen tragen.

12. Test-Kit nach Anspruch 11, dadurch gekennzeichnet, daß ein Feld Adenin trägt, und das zweite Feld Histidin trägt.

13. Test-Kit nach Anspruch 10, dadurch gekennzeichnet, daß auf dem Trägermaterial drei Felder markiert sind, die unterschiedliche Substanzen aus der Gruppe der Aminosäuren und der Purine oder aus beiden Gruppen tragen.

14. Test-Kit nach einem der Ansprüche 10 bis 13, dadurch gekennzeichnet, daß die Felder zusätzlich farblich unterschiedlich getönt sind.

15. Test-Kit nach Anspruch 10 oder 14, dadurch gekennzeichnet, daß auf dem Trägermaterial oder benachbart zu dem Trägermaterial zusätzlich eine Farbvergleichsskala mit unterschiedlichen Hautfarbtönen angeordnet ist.

16. Test-Kit nach einem der Ansprüche 10 bis 15, dadurch gekennzeichnet, daß das Testfeld mit einer entfernbaren Folie abgedeckt ist.
